# EUROPÄISCHE PATENTSCHRIFT

(11) **EP 1 077 688 B1**
(45) Veröffentlichungstag und Bekanntmachung des Hinweises auf die Patenterteilung: **12.12.2001**
(21) Anmeldenummer: 99923585.6
(22) Anmeldetag: 12.05.1999
(51) Int. Cl.: A61K 9/70, A61K 31/48

(54) **TRANSDERMALES THERAPEUTISCHES SYSTEM (TTS) PERGOLID ENTHALTEND**
TRANSDERMAL THERAPEUTIC SYSTEM CONTAINING PERGOLIDE
SYSTEME THERAPEUTIQUE TRANSDERMIQUE CONTENANT DU PERGOLIDE

(30) Priorität: 15.05.1998 DE 19821788
(43) Veröffentlichungstag der Anmeldung: 28.02.2001
(73) Patentinhaber: SCHWARZ PHARMA AG, 40789 Monheim (DE)
(72) Erfinder: ARTH, Christoph, D-40593 Düsseldorf (DE); KOLLMEYER-SEEGER, Andreas, D-40764 Langenfeld (DE); RIMPLER, Stephan, D-40723 Hilden (DE); WOLFF, Hans-Michael, D-40789 Monheim (DE)
(86) Internationale Anmeldenummer: EP9903278
(87) Internationale Veröffentlichungsnummer: WO9959558

(56) Entgegenhaltungen:
- WO-A-96/40139
- WO-A-99/01116
- DE-A- 4 310 012
- DE-A- 19 626 621

## Beschreibung

Die vorliegende Erfindung betrifft ein Transdermales Therapeutisches System (TTS) zur transcutanen Verabreichung von Pergolid über mehrere Tage sowie ein Verfahren zu seiner Herstellung ohne Verwendung von Lösungsmitteln.

Die Bioverfügbarkeit von oral verabreichten Wirkstoffen ist oft unbefriedigend. Die hepatische Metabolisierung vieler Wirkstoffe kann bei der ersten Leberpassage zu unerwünschten Konzentrationsverhältnissen, toxischen Nebenprodukten und zur Verminderung der Wirkung oder gar zum Wirkverlust führen. Gegenüber oraler Verabreichung besitzt die transdermale Gabe von Wirkstoffen verschiedene Vorteile. Die Wirkstoffzufuhr läßt sich über einen längeren Zeitraum besser steuem, wodurch hohe Blutspiegelschwankungen vermieden werden. Zudem kann die erforderliche therapeutisch wirksame Dosis meist deutlich verringert werden. Außerdem wird ein Pflaster vom Patienten oft mehr bevorzugt als täglich einmal oder mehrfach einzunehmende Tabletten.

In der Vergangenheit wurde zur Überwindung der vorgenannten Nachteile der nichttransdermalen Gabe von Wirkstoffen durch eine Vielzahl von Transdermalen Therapeutischen Systemen (TTS) mit unterschiedlichem Aufbau für verschiedene Wirkstoffe zur Therapie unterschiedlicher Erkrankungen Rechnung getragen.

So beschreiben die nachfolgend genannten technischen Dokumente für eine breite Vielfalt systemisch oder lokal reagierender Wirkstoffe deren parenterale Verabreichung entweder auf Basis dosis-kontrollierender oder allgemein freisetzender Systeme. Beispielhaft sind dies: U.S.P.
3,598,122; 3,598,123; 3,731,683; 3,797,494; 4,031,894; 4,201,211; 4,286,592; 4,314,557; 4,379,454; 4,435,180; 4,559,222; 4,568,343; 4,573,995, 4,588,580; 4,645,502; 4,702,282; 4,788,062; 4,816,258; 4,849,226; 4,908,027; 4,943,435 und 5,004,610.

In den späten sechziger Jahren dieses Jahrhunderts war ursprünglich theoretisch angenommen worden, daß jeder Wirkstoff mit kurzer Halbwertszeit aber hoher Wirksamkeit und guter Hautdurchgängigkeit für eine sichere und effektive Verabreichung mittels eines TTS geeignet sei. Diese anfänglichen Erwartungen hinsichtlich der Möglichkeiten der transdermalen Verabreichung von Wirkstoffen mittels TTS konnten jedoch nicht erfüllt werden. Dies findet seine Begründung hauptsächlich darin, daß die Haut von Natur aus mit einer unüberschaubaren Vielfalt von Eigenschaften ausgestattet ist, um ihre Funktion als intakte Barriere gegenüber dem Eindringen von nicht-körpereigenen Substanzen in den Körper aufrecht zu erhalten. (Siehe hierzu: Transdermal Drug Delivery: Problems and Possibilities, B.M. Knepp et al., CRC Critical Review and Therapeutic Drug Carrier Systems, Vol. 4, Issue 1 (1987)).

Daher steht die transdermale Verabreichung nur für diejenigen wenigen Wirkstoffe zur Verfügung, die eine geeignete Kombination von vielen günstigen Charakteristika aufweisen. Für einen bestimmten Wirkstoff sind diese geforderten Charakteristika, die die sichere und effektive transdermale Verabreichung gewährleisten sollen, jedoch nicht vorhersagbar.

Die an einen für die transdermale Verabreichung geeigneten Wirkstoff zu stellenden Anforderungen sind:
- Hautdurchgängigkeit,
- keine Beeinträchtigung des Klebevermögens des Pflasters durch den Wirkstoff,
- Vermeidung von Hautirritationen,
- Vermeidung von allergischen Reaktionen,
- günstige pharmakokinetische Eigenschaften,
- günstige pharmakodynamische Eigenschaften,
- ein relativ weites therapeutisches Fenster,
- Metabolismuseigenschaften, die konsistent mit der therapeutischen Anwendung bei kontinuierlicher Gabe sind.

Unzweifelhaft ist die vorgenannte Liste der Anforderungen nicht erschöpfend. Damit ein Wirkstoff für die transdermale Verabreichung zur Verfügung stehen kann, ist die "richtige" Kombination all dieser Anforderungen wünschenswert.

Das für die Wirkstoffe vorgenannte gilt in gleicher Weise für die den jeweiligen Wirkstoff enthaltende TTS-Zusammensetzung und deren konstruktiven Aufbau.

Üblicherweise handelt es sich bei den Transdermalen Therapeutischen Systemen (TTS) um Pflaster, die mit einer undurchlässigen Deckschicht, einer abziehbaren Schutzschicht und einer wirkstoffhaltigen Matrix oder einem wirkstoffhaitigen Reservoir mit semipermeabler Membran ausgestattet sind. Im ersten Fall werden sie als Matrixpflaster, im zweiten Fall als Membransystem bezeichnet.

Für die Deckschicht werden üblicherweise Polyester, Polypropylen, Polyethylen, Polyurethan etc. verwendet, die auch metallisiert oder pigmentiert sein können. Für die abziehbare Schutzschicht kommen u.a. Polyester, Polypropylen oder auch Papier mit Silikon- und/oder Polyethylenbeschichtung in Betracht.

Für die pharmazeutisch bzw. medizinisch üblichen wirkstoffhaltigen Matrices werden Stoffe auf Basis von Polyacrylat, Silikon, Polyisobutylen, Butylkautschuk, Styrol/ Butadien-Copolymerisat oder Styrol/Isopren-Copolymerisat verwendet.

Die in Membransystemen verwendeten Membranen können mikroporös oder semipermeabel sein und werden üblicherweise auf Basis eines inerten Polymeren, insbesondere Polypropylen, Polyvinylacetat oder Silikon gebildet.

Während die wirkstoffhaltigen Matrixzusammensetzungen selbstklebend sein können, ergeben sich aber auch in Abhängigkeit von eingesetztem Wirkstoff wirkstoffhaltige Matrices, die nicht selbstklebend sind, so daß als Folge hiervon das Pflaster oder TTS konstruktiv mit einem Overtape versehen werden muß.

Zur Sicherstellung der erforderlichen Fluxrate des Wirkstoffes sind häufig Hautpenetrationsenhancer wie aliphatische, cycloaliphatische und/oder aromatisch-aliphatische Alkohole, jeweils ein- oder mehrwertig und jeweils mit bis zu 8 C-Atomen umfassend, ein Alkohol-/Wasser-Gemisch, ein gesättigter und/oder ungesättigter Fettalkohol mit jeweils 8 bis 18 Kohlenstoffatomen, eine gesättigte und/oder ungesättigte Fettsäure mit jeweils 8 bis 18 Kohlenstoffatomen und/oder deren Ester sowie Vitamine als Zusatz erforderlich.

Weiterhin werden häufig Stabilisatoren, wie Polyvinylpyrrolidon, α-Tocopherolsuccinat, Propylgallat, Methionin, Cystein und/oder Cystein-hydrochlorid, der wirkstoffhaltigen Matrix zugesetzt.

Wie die vorgenannte Aufstellung zeigt, sind zahlreiche TTS-Konstruktionen und hierfür verwendete Materialien bekannt. Allerdings sind viele interagierende Erfordernisse zu berücksichtigen, wenn ein Medikament in Form eines TTS einem medizinischen Bedürfnis genügen soll.

Die folgenden Problemstellungen sind bei der Entwicklung von wirkstoffhaltigen TTS zu berücksichtigen:
1. Zum Erreichen therapeutisch notwendiger Penetrationsraten des Wirkstoffes durch die Haut ist meist eine hohe Wirkstoffbeladung der Polymermatrix erforderlich. Nach Applikationsende im TTS verbleibender Wirkstoff wird therapeutisch ungenutzt mit dem Pflaster entsorgt. Dies ist jedoch, insbesondere bei hochwirksamen und teuren Wirkstoffen, aus Umweltschutz- und Kostengründen unerwünscht.
2. Die wirkstoffbeladene und ggf. zusätzlich mit Hautpenetrationsenhancem beladene Polymermatrix ist bei längerer Lagerung physikalisch nicht stabil. Insbesondere kann eine Wirkstoffrekristallisation auftreten, die zu einer nicht kontrollierbaren Abnahme der Wirkstofffreisetzungskapazität des TTS führt.
3. Eine hohe Beladung des polymeren Trägerstoffes mit Wirkstoff und/oder Hautpenetrationsenhancem erschwert bei selbstklebenden Polymerfilmen die Einstellung optimaler Hafteigenschaften des transdermalen Systems.
4. Die Wirkstoffresorptionsrate sinkt bei Anwendungen über mehrere Tage in nicht akzeptabler Weise ab, so daß zusätzliche Steuerschichten und/oder -komponenten erforderlich sind.
5. Werden wirkstoffbeladene Schichten aus organischen Lösungen hergestellt, tritt das Problem des Verbleibens von Lösemittelresten in der wirkstoffhaltigen Schicht nach dem Trocknungsprozeß auf. Zusätzlich besteht die Gefahr einer unerwünschten Verdunstung von flüchtigen Hilfsstoffen während der Herstellung. Da aus Gründen der physikalischen Stabilität und Hautverträglichkeit des Systems in der Regel vollständige Lösungsmittelfreiheit anzustreben ist, muß das Reservoir gegebenenfalls in mehreren Schichten aufgebaut werden. Dies wiederum führt zu einer Erhöhung der Herstellungskosten.

Die beschriebenen Probleme bedingen daher eine Vielzahl von Ausführungsformen Transdermaler Therapeutischer Systeme, die sich im Stand der Technik auf diesem Gebiet widerspiegeln.

Eine neuere Übersicht hierzu gibt beispielsweise **U.S. P 5,662,926** (Wick et al., 1997). Dieses Dokument beschreibt transdermale Systeme, die einen monolithischen, thermoplastischen Polymerfilm enthalten, in dem ein Wirkstoff, vorzugsweise Nikotin, homogen verteilt ist, sowie ein Verfahren zur lösungsmittelfreien Herstellung dieser wirkstoffhaltigen Schicht durch Mischen des wirksamen Bestandteils mit dem polymeren Trägermaterial in der Polymerschmelze bei Temperaturen von 170°C bis 200°C. Zur Fixierung des wirkstoffhaltigen Matrixfilms auf der Haut dient ein zusätzlicher Kontaktkleberfilm, der auf die Wirkstoffmatrix aufgebracht wird und, falls erforderlich, zusätzlich ein flächengrößeres Pflaster, das auf der von der Haut abgewandten Matrixseite auf den wirkstoffhaltigen Polymerfilm aufgebracht wird.

Ähnliche Aufbauprinzipien für transdermale Systeme oder Wirkstoffpflaster sind auch in PCT/US96/09692 und DE 196 26 621 für Pergolid-haltige Pflasterzubereitungen beschrieben. Gemäß beiden Dokumenten kann die Hautpenetration von Pergolid aus den Polymermatrices durch spezielle Penetrationsenhancer erhöht werden. in PCT/US96/09692 werden die als Wirkstoffträger verwendeten Ethylen-Vinylacetat(EVA)-Copolymere zur Einarbeitung von Pergolid in einem geeigneten organischen Lösungsmittel aufgelöst In DE 196 26 621 wird Pergolid in die als Wirkstoffträger verwendeten Acrylatpolymere eingearbeitet, indem der Wirkstoff in einer Lösung der Polymere in organischen Lösungsmitteln dispergiert wird. Die Erzeugung von Filmen erfolgt jeweils anschließend durch Beschichten und Enfternung des organischen Lösungsmittels aus der entsprechenden Polymer-/Wirkstoff-/Penetrationsenhancermischung.

Zur Behandlung des Morbus Parkinson sind gemäß **PCT/US96/09692** Pergolid-Plasmaspiegel in der Größenordnung von 0,1-1 ng/ml anzustreben, entsprechend einer Freisetzungsrate von mindestens 100 µg/h vorzugsweise 150 µg/h.

In der Entwicklung von transdermalen Systemen sind Polymere auf Acrylsäureester und Methacrylsäureesterbasis von besonderem Interesse wegen ihres relativ guten Aufnahme- und Abgabevermögens für eine Vielzahl von Wirkstoffen. Um die Verwendung von Lösungsmitteln bei der Herstellung von Matrixsystemen auf Poly(meth)acrylatbasis zu vermeiden, ist in **DE 4310012** ein dermales therapeutisches System beschrieben, in dem eine oder mehrere Schichten aus Mischungen von Poly(meth)acrylaten aufgebaut und aus der Schmelze hergestellt werden und die erste Mischungskomponente aus (Meth)acrylpolymeren besteht, die funktionelle Gruppen enthalten, die zweite Mischungskomponente das Fließverhalten reguliert und nur unerhebliche Mengen an funktionellen Gruppen enthält. Die zusammengesetzten Systeme mit Poly(meth)acrylaten mit funktionellen Gruppen sollen eine gesteuerte Abgabe des oder der Wirkstoffe an bzw. durch die Haut und eine einfache Art der Herstellung ermöglichen. Den Vorteilen in der Herstellung gegenüber lösungsmittelbasierten Verfahren stehen bei solchen Systemen jedoch erfahrungsgemäß eine Reihe von Nachteilen gegenüber, die bedingt sind durch:
1. Länger andauernde thermische Belastung aller TTS-Komponenten bei (1) der Herstellung der Polymerschmelze, (2) der homogenen Einarbeitung des oder der Wirkstoffe und/oder (3) der Beschichtung der heißen wirkstoffhaltigen Masse auf geeignete Trägermaterialien mit erhöhtem Risiko von Abbau- bzw. Zersetzungsreaktionen in der Polymerschmelze und/oder während der Lagerung der wirkstoffhaltigen Polymerfilme.
2. Schwierigkeiten in der Optimierung der Co-/Adhäsionsbalance der poly(meth)acrylathaltigen Schicht, da eine Vernetzung des Acrylatcopolymerisates mittels kovalenter Bindungen während der Herstellung der wirkstoffhaltigen Polymermatrix in der Schmelze nicht möglich ist, verbunden mit Problemen, die durch einen kalten Fluß der Polymermasse bei Anwendung auf der Haut und/oder bei Lagerung auftreten können.

Wie die vorgenannte Aufstellung zeigt, sind viele Pflasterkonstruktionen und hierfür verwendete Materialien bekannt. Gleichwohl besteht bis heute für viele in Transdermalen Therapeutischen Systemen verarbeitete Wirkstoffe ein großer Bedarf, TTS zur Verfügung zu stellen, die eine therapeutisch geforderte Wirkstoffabgabe ermöglichen, ohne dabei konstruktiv aufwendig zu sein und in der Gesamtschau ihrer Bestandteile eine optimale Beziehung darstellen. Dies gilt auch für den Wirkstoff Pergolid, wenn er transcutan verabreicht werden soll.

Therapeutisch wird Pergolid allein oder in Kombination mit weiteren Wirkstoffen zur Behandlung des Morbus Parkinson eingesetzt. Die transcutane Applikation von Pergolid mittels eines TTS ist wünschenswert, da unter Umgehung des Magen-Darm-Traktes und der ersten Leberpassage Konzentrationsspitzen von Pergolid im Blut vermieden werden, die zum Auftreten unerwünschter Wirkungen wie Halluzinationen, Schwindel, Übelkeit und Erbrechen, führen können. Durch Umgehung des "first-pass"-Metabolismus in der Leber kann gegenüber der peroralen Gabe die Bioverfügbarkeit von Pergolid erhöht werden, bzw. die Gesamtdosis reduziert werden, die zum Erreichen der therapeutisch gewünschten Wirkung erforderlich sind.

Aufgabe der vorliegenden Erfindung ist es daher, die vorgenannten Nachteile der TTS mit Pergolid zu vermeiden und ein konstruktiv einfaches, hautverträgliches, über eine längere Lagerungs- und Applikationsdauer physikalisch und chemisch stabiles TTS zur transcutanen Verabreichung von Pergolid zur Verfügung zu stellen, das
a) bei geringer Wirkstoffbeladung pro Flächeneinheit möglichst viel Wirkstoff an und durch die Haut freisetzt,
b) nach Applikationsende den enthaltenen Wirkstoff möglichst vollständig an die Haut abgegeben hat,
   und
c) lösemittelfrei ist.

Zur Lösung dieser Aufgabe wird ein TTS und ein Verfahren zu seiner Herstellung ohne Verwendung von Lösemitteln zur Verfügung gestellt, dessen besondere Zusammensetzung überraschenderweise der vorgenannten Aufgabenstellung genügt. Es enthält eine Pergolid-haltige Matrixmasse in Form einer Schicht, wobei die Matrixmasse ein ammoniogruppenhaltiges (Meth)acrylatcopolymer oder eine Mischung aus aminogruppenhaltigem (Meth)acrylatcopolymeren und carboxylgruppenhaltigem (Meth)acrylatpolymeren, 10 bis 50 Gew.-% Propylenglykol, bis zu 5,0 Gew.-% Pergolid oder ein pharmazeutisch unbedenkliches Salz hiervon (berechnet als Base) enthält und dieses, mit Ausnahme seiner Freisetzungsfläche an der Applikationsstelle von einem größeren wirkstofffreien Pflaster zur Fixierung an der Haut umgeben ist.

Im Sinne der Erfindung werden die nachfolgend genannten Begriffe und/oder Worte, wie folgt, verstanden:
- a) "Iösemittelfrei":: zur Herstellung der Polymermatrices werden keine Lösungsmittel verwendet, die im Verlaufe des Herstellungsverfahrens wieder weitgehend entfernt werden, wie dieses im sogenannten "solvent based"-Verfahren geschieht.
- b) "mehrere Tage":: die TTS können zur therapeutischen Anwendung von 1 bis zu 3 Tagen auf die Haut appliziert werden.
- c) "feste Lösung":: der pharmazeutische Wirkstoff liegt in der Pflastermatrix molekulardispers verteilt vor.

Aufgrund der erfindungsgemäßen Zusammensetzung und des konstruktiven Aufbaus des TTS ist es überraschend, daß hohe Anteile an Propylenglykol in der Polymermatrix stabil inkorporiert werden können, ohne daß Propylenglykol bei Langzeitlagerung aus der Pflastermatrix ausblutet.

Nach einer bevorzugten Ausführungsform sind in der Polymermatrix 0,5 bis 2 Gew. % Pergolid oder ein pharmazeutisch unbedenklichesSalz hiervon (berechnet als Base) enthalten.

Pergolid kann im TTS als freie Base oder in Form eines seiner pharmazeutisch verwendbaren Säureadditionssalze wie z.B. Pergolid-hydrochlorid, -acetat oder -mesylat in der Polymermatrix enthalten sein. Bevorzugt ist in der Polymermatrix Pergolidmesylat enthalten.

Nach einer weiteren Ausführungsform enthält die Pergolid-haltige Matrixmasse einen oder mehrere lipophile Hautpenetrationsenhancer und/oder einen oder mehrere Weichmacher.

Geeignete Hautpenetrationsenhancer sind beispielsweise gesättigte und/oder ungesättigte Fettsäuren mit jeweils 8 bis 18 Kohlenstoffatomen, deren Ester mit ein- oder mehrwertigen aliphatischen Alkoholen oder gesättigten und/oder ungesättigte Fettalkohole mit jeweils 8 bis 18 Kohlenstoffatomen.

Bevorzugt ist in der Pergolid-haltigen Matrixmasse Propylenglykolmonolaurat als Hautpenetrationsenhancer enthalten.

Geeignete Weichmacher sind beispielsweise Triester der Zitronensäure mit Alkanolen mit jeweils 1-4 Kohlenstoffatomen, Triester von Glycerol mit Alkansäuren mit jeweils 1-4 Kohlenstoffatomen, Diester der Phthalsäure mit Alkanolen mit jeweils 1-4 Kohlenstoffatomen und Polyoxyethylen-Polyoxypropylen-Copolymere mit einer Molekularmasse zwischen 5.000 und 10.000.

Bevorzugt enthaltene Weichmacher sind Zitronensäuretributylester oder/und Zitronensäuretriethylester.

Nach einer weiteren Ausführungsform der Erfindung weist die Trägerfolie des TTS matrixseitig eine Metalldampf- oder Oxidbeschichtung auf.

Das erfindungsgemäße TTS kann nach dem nachfolgend erläuterten Verfahren hergestellt werden.

Eine beschichtungsfähige Pergolid-haltige Matrixmasse wird durch Schmelzextrusion erzeugt, wobei homogene, bis zu 150°C heiße Polymerschmelze, bestehend aus ammoniogruppenhaltigem (Meth)acrylatcopolymeren oder einer Mischung aus aminogruppenhaltigem (Meth)acrylatcopolymeren mit carboxylgruppenhaltigem (Meth)acrylatpolymeren, 10 bis 50 Gew.-% Propylenglykol, bis zu 5,0 Gew.-% Pergolid oder einem pharmazeutisch unbedenklichen Salz hiervon (berechnet als Pergolidbase) sowie gegebenenfalls einem oder mehreren Hautpenetrationsenhancer/n und/oder einem oder mehreren Weichmacher/n kontinuierlich in einer Dicke von 0,02 - 0,4 mm auf einen Träger beschichtet wird, das erhaltene 2-Schichtlaminat mit einer Deckschicht versehen wird und hierauf ein größeres wirkstofffreies Pflaster zur Fixierung des TTS auf der Haut aufgebracht wird.

Der wesentliche Vorteil des erfindungsgemäßen Verfahrens im Gegensatz zum sogenannten "batch-Verfahren" besteht darin, daß die den Wirkstoff enthaltende Polymermatrix (I) ohne Verwendung von organischen Lösungsmitteln hergestellt wird, und (II) die Zubereitung der wirkstoffhaltigen Matrixmasse und ihre weitere Verarbeitung zu einer wirkstoffhaltigen Schicht in einem kontinuierlichen und kosteneinsparenden Arbeitsgang erfolgen: Prozeßzeiten lassen sich auf wenige Minuten verkürzen. Die Gefahr von Zersetzungsreaktionen in der wirkstoffhaltigen Polymerschmelze kann hierdurch insoweit ausgeschlossen werden.

Ferner werden durch die kontinuierliche Herstellung der Pergolid-haltigen Polymermasse Scaling-Up Probleme umgangen, d.h. bei Erhöhung der Ansatz- bzw. Chargengröße ist zur Herstellung der wirkstoffhaltigen Polymerschmelze und des Laminates kein Wechsel auf größere Produktionsanlagen erforderlich, der üblicherweise mit zeit- und kostenaufwendigen Installations-, Qualifizierungs- und Validierungsarbeiten sowie ggf. auch Rezepturänderungen verbunden ist.

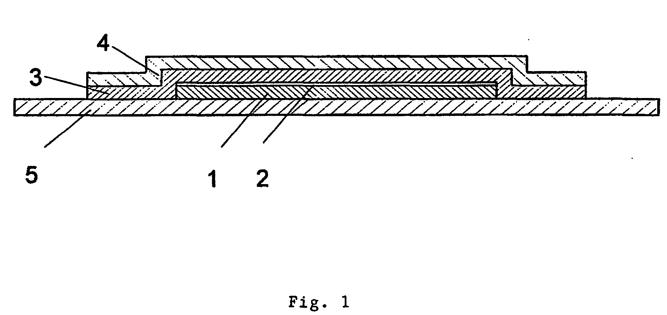

Der Aufbau der erfindungsgemäßen TTS ist in Zeichnung 1 dargestellt.

Es besteht aus einer wirkstoffhaltigen Polymermatrix (1), einer ablösbaren Schutzfolie (5), einer inneren Deckfolie(2) sowie einem Overtape, bestehend aus Trägerfolie (4) und Adhäsivfilm (3).

### Die Erfindung wird anhand folgender Beispiele erläuert:

### Beispiele 1 bis 4

Ein mit 2 Dosierem ausgerüsteter gleichsinnig laufender Doppelschneckenextruder wird kontinuierlich in zwei aufeinanderfolgenden Verfahrenszonen mit einer festen Komponente bzw. homogenen Feststoffmischung (Komponente A) sowie einer flüssigen Komponente B beschickt (zur Zusammensetzung der Komponenten A und B siehe Tabelle 1). Der Ansatz wird mit einem Gesamtdurchsatz von 1 kg/h bei einer Temperatur von 140°C schmelzextrudiert, wobei aus Dosierer 1 die Komponente A in das erste Verfahrensteil und aus Flüssigdosierer 2 die Komponente bzw. Lösung B in das zweite Verfahrensteil kontinuierlich zugewogen werden (Dosierraten siehe Tabelle 2). Nach dem Verlassen des Extruders wird die erhaltene heiße Pergolid-haltige Polymerschmelze direkt auf eine ca. 100 µm dicke Polyesterfolie (= Schutzfolie (5)) so beschichtet, daß das Auftragsgewicht der Polymermasse ca. 50 g/m² beträgt. Das Zweischichtiaminat, bestehend aus Schutzfolie und Matrixmasse, wird nach Abkühlen mit einer ca. 20 µm dicken Polyesterfolie (= innere Deckfolie (2)) abgedeckt.

Aus dem erhaltenen bahnförmigen Dreischichtlaminat werden die Konturen 5 cm² großer Matrixstücke ausgestanzt, wobei die innere Deckfolie (2) und wirkstoffhaltige Polymermatrix (1), nicht aber die Schutzfolie (5) durchtrennt werden. Die entstehenden Zwischenstege werden abgegittert. Auf das erhaltene bahnförmige Laminat mit formatgestanzten TTS-Matrices wird eine 2-schichtig aufgebaute, selbstklebende Overtapefolie, bestehend aus einem Haftkleberfilm (3) auf Basis eines vemetzten Acrylatcopolymeren und einer (äußeren) Trägerfolie (4) aus Polyurethan, aufkaschiert. Das resultierende Laminat wird zu 20 cm² großen Pflastern, bestehend aus den Komponenten (1), (2), (3), (4), (5) entsprechend Zeichnung 1 ausgestanzt.

**Tabelle 1:**

| Herstellformel der Beispiele 1 bis 4 | | | | |
|---|---|---|---|---|
| Bestandteil | Beispiel 1 Gew.-% | Beispiel 2 Gew.-% | Beispiel 3 Gew.-% | Beispiel 4 Gew.-% |
| **Komponente A (fest)** | | | | |
| Pergolidmesilat | 1,67 | 1,74 | 1,47 | ./. |
| Eudragit 4135 F¹⁾ | 13,33 | 13,91 | ./. | 13,56 |
| Eudragit E 100²⁾ | 85,00 | 84,35 | ./. | 86,44 |
| Eudragit RS 100³⁾ | ./. | ./. | 98,53 | ./. |

| **Komponente B (flüssig)** | | | | |
|---|---|---|---|---|
| Propylenglykol (PG) | 100,00 | 94,12 | 100,00 | ./. |
| Propylenglykolmonolaurat | ./. | 5,88 | ./. | ./. |
| 2,5 % P.-Mesilatlösung in PG | ./. | ./. | ./. | 100,00 |

1) Copolymer aus Methacrylsäure, Methylacrylat und Methylmethylacrylat; entspricht dem polymeren Bestandteil der wäßrigen Dispersion Eudragit 4110 D gemäß Technischem Merkblatt Präparat Eudragit 4110 D, 05/97, Fa. Röhm, Darmstadt, Deutschland
2) Copolymer aus Dimethylaminoethylmethylacrylat und neutralen Methacrylsäureestem mit Ameisen- und Buttersäure; entspricht Normenblatt Eudragit 100, 01/96, Fa. Röhm, Darmstadt, Deutschland
3) Copolymer aus Acryl- und Methacrylsäureestem, entsprechend "Ammino Methacrylate Copolymer" Type B gemäß USP 23/NF 18

**Tabelle 2:**

| Herstellparameter (Dosierraten) zu den Beispielen 1 bis 4 | | | | |
|---|---|---|---|---|
| | Dosierrate (g/h) | | | |
| | Bsp. 1 | Bsp. 2 | Bsp. 3 | Bsp. 4 |
| Feststoffdosierung (Komponente A) | 600 | 575 | 680 | 1.180 |
| Feststoffdosierung (Komponente B) | 400 | 425 | 320 | 820 |

### Fluxmessungen des Pergolid in vitro

### a) Fluxmessungen durch Mäusehaut

Eine TTS-Matrix mit einer ausgestanzten Fläche von 2,5 cm² wird in einer horizontalen Diffusionszelle auf die Homschichtseite der Bauch- und Rückenhaut haarloser Mäuse fixiert. Unmittelbar anschließend wird die Akzeptorkammer der Zelle mit auf 32°C vortemperierter Phosphat-Pufferlösung pH 6,2 (Ph. Eur., pH 6,4 R; mit Phosphorsäure auf pH 6,2 eingestellt) luftblasenfrei befüllt und das Freisetzungsmedium auf 32 ± 0,5 °C thermostatisiert.

Zu den Probeentnahmen (nach 3; 6; 24; 30; 48; 54 und 72 Stunden) wird das Freisetzungsmedium gegen frisches, auf 32 ± 0,5 °C thermostatisiertes Medium ausgetauscht.

Der Gehalt an Pergolidmesilat im Freisetzungs- bzw. Akzeptormedium wird mittels Hochdruckflüssigkeitschromatographie unter den nachfolgend aufgeführten Bedingungen bestimmt. Stationäre Phase: Supelcosil LC-8-DB, 75 mm x 4,6 mm, 3 µm; 45°C; Säulentemperatur: 40°C; Eluent: 550 Volumenteile Wasser, 450 Volumenteile Methanol und 1,7 Volumenteile Dibutylamin, eingestellt auf pH 3,0 mit Phosphorsäure; Detektion: Fluoreszenz, Anregungswellenlänge = 280 nm, Emissionswellenlänge = 346 nm; Flußrate: 1,5 ml/min.; Injektionsvolumen: 25 µl.

### b) Fluxmessungen durch Humanhaut

Verwendet wurde eine max. 8 Wochen bei -18°C gelagerte Hautprobe aus dem Bauchbereich einer Frau. Die Präparation der für die Fluxmessungen eingesetzten Hautstücke erfolgte durch Abtrennung der Dermis mittels Hitze-Separation (Klingman & Christopher, 88, Arch. Dermatol. 702 (1963)) in 60°C warmem Wasser, die erhaltene epidermale Membran wurde auf Filterpapier bei -18°C über max. 1 Woche aufbewahrt und vor Durchführung der Messung über Nacht aufgetaut.

Die TTS-Matrix wurde in einer modifizierten Franz-Zelle mit einer Freisetzungs- bzw. Diffusionsfläche von 1 cm² auf die Homschichtseite der exzidierten Hautpräparation fixiert. Unmittelbar anschließend wurde Akzeptorkammer der Zelle (2,3 cm³ Volumen) mit Phosphat-Pufferlösung pH 6,2 (Ph. Eur., pH 6,4 R; mit Phosphorsäure auf pH 6,2 eingestellt) luftblasenfrei befüllt und das Freisetzungsmedium auf 37 ± 0,5 °C thermostatisiert (entsprechend einer Hautoberflächentemperatur in der verwendeten Diffusionszelle von 32°C).

Zu den Probeentnahmezeiten (nach 3; 6; 9; 12; 24; 36; 48 Stunden) wurde das Freisetzungsmedium gegen frisches, vortemperiertes Medium ausgetauscht.

Der Gehalt an Pergolidmesilat in dem Freisetzungs- bzw. Akzeptormedium wird mittels Hochdruckflüssigkeitschromatographie unter den nachfolgend aufgeführten Bedingungen bestimmt. Stationäre Phase: Supelcosil LC-8-DB, 150 mm x 4,3 mm, 3µm; 45°C; Säulentemperatur: 40°C; Eluent: 55 Volumenteile Wasser, 45 Volumenteile Methanol und 1,7 Volumenteile Dibutylamin, eingestellt auf pH 3,0 mit Phosphorsäure; Detektion UV bei 280 nm; Flußrate: 1,0 ml/min.; Injektionsvolumen: 20 µl.

Die Ergebnisse der Untersuchungen an Prüfmustem gemäß den Beispielen 1 bis 3 sind in Tabelle 3 zusammengestellt. Tabelle 4 enthält eine Übersicht der Fluxraten von aus dem Stand der Technik bekannten Polymermatrixsystemen bzw. Lösungen gemäß PCT/US96/09692, welche einen Massenanteil an Pergolid von 5 - 10 Gew.-% bzw. 2 und 5 Gew.-% aufweisen.

Ein Vergleich der Fluxraten zeigt, daß das erfindungsgemäße TTS trotz einer mit einem Massenanteil von weniger als 1 Gew.-% deutlich geringeren Wirkstoffbeladung als in den Vergleichsbeispielen Pergolid in überraschend hohen Raten durch die Haut freisetzt. So weist die im erfindungsgemäßen TTS enthaltene Matrix entsprechend Beispiel 2, welche einen Wirkstoffanteil von nur 0,87 Gew.-% enthält, mit 2,4 µg/cm²/h sogar einen höheren Flux durch Humanhaut auf als sämtliche Matrixformulierungen von PCT/US96/ 09692, welche einen Pergolidanteil von bis zu 10 Gew.-% sowie hohe Anteile an Hautpenetrationsenhancem enthalten. Dieses Ergebnis ist umso überraschender, als daß die Fluxmessung mit einem auf 32°C temperierten Akzeptormedium überdies bei einer um 3°C niedrigeren Temperatur als in PCT/US96/09692 durchgeführt wurde, also unter Bedingungen, welche für die Wirkstoffpenetration deutlich ungünstiger sind.

Darüber hinaus zeigt sich, daß die im erfindungsgemäßen TTS enthaltene Pergolidmenge im Untersuchungszeitraum von 2 Tagen praktisch quantitativ durch die Haut freigesetzt werden kann. Dies ist besonders vorteilhaft, da hierdurch nach Applikationsende im TTS verbleibende Restmengen des hochwirksamen und teuren Wirkstoffes vermieden werden können.

**Tabelle 3:**

| Pergolid-Fluxraten durch exzidierte Hautpräparationen (Beispiele 1-3) | | | | |
|---|---|---|---|---|
| Präparat | Pergolidmesilatgehalt der Matrix Gew.-% (mg/16cm²) | Fluxrate (µg/cm²/h) | Mittlere kumulative Fluxrate (µg/12cm²) | |
| | | | Nach 24 h | Nach 48 h |
| | | Mäusehaut | | |
| Beispiel 1: | 0,61 % (0,48 mg ± 10 %) | 1,0 | 394 (82 %)* | 454 (95 %)* |
| Beispiel 2: n=3 | 0,87 % (0,78 mg ± 10 %) | 1,8 | 687 (88 %)* | 815 (ca.100 %)* |

| | | Humanhaut | | |
|---|---|---|---|---|
| Beispiel 1: | 0,61 % (0,48 mg ± 10 %) | 1,0 | 258 (54 %)* | 302 (63 %)* |
| Beispiel 2: n=4 | 0,87 % (0,78 mg ± 10 %) | 2,4 | 576 (74 %)* | 682 (87%)* |

| | | Mäusehaut | | |
|---|---|---|---|---|
| Beispiel 3: n=3 | 0,67 % (0,71 mg ± 10 %) | 1,5 | 559 (79 %)* | 609 (86%)* |

| | | | | |
|---|---|---|---|---|
| *) = kumulativer Flux in Gew.-% bezogen auf den jeweils angegebenen Wirkstoffgehalt der Matrix | | | | |

**Tabelle 4:**

| Pergolid-Fluxraten durch exzidierte Hautpräparationen (PCT/US96/09692) | | |
|---|---|---|
| Präparat | Pergolidmesilagehalt von Matrix bzw. Donor Gew.-% | Fluxrate* µg/cm²/h |
| (1) P.-mesilatlösung in H₂O | 2 | ca. 1,1 |
| (2) P.-mesilatlösung in H₂O/Ethanol | 5 | ca. 2 - 4 |
| (3) P.-mesilat in EVA-Polymermatrix | 5 - 10 | 0,5 - 2,2 |

| | | |
|---|---|---|
| *) = Prüfungen an exzidierter Humanhaut; 35 °C | | |

## Patentansprüche

1. Transdermales therapeutisches System (TTS) zur transcutanen Verabreichung von Pergolid über mehrere Tage mit einer Fixierungshilfe für das TTS auf der Haut, **dadurch gekennzeichnet, daß** das TTS eine schichtförmige Pergolid-haltige Matrixmasse enthält, die ein ammoniogruppenhaltiges (Meth)acrylatcopolymer oder eine Mischung aus aminogruppenhaltigem (Meth)acrylatcopolymeren und carboxylgruppenhaltigem (Meth)acrylatpolymeren, 10 bis 50 Gew.-% Propylenglykol, bis zu 5,0 Gew.-% Pergolid oder ein pharmazeutisch unbedenkliches Salz hiervon (berechnet als Base) enthält und dieses, mit Ausnahme seiner Freisetzungsfläche an der Applikationsstelle von einem größeren wirkstofffreien Pflaster zur Fixierung an der Haut umgeben ist.

2. TTS nach Anspruch 1, **dadurch gekennzeichnet, daß** die wirkstoffhaltige Matrixmasse 0,5 bis 2 Gew.-% Pergolid oder ein pharmazeutisch unbedenkliches Salz hiervon (berechnet als Base) enthält.

3. TTS nach Ansprüchen 1 - 2, **dadurch gekennzeichnet, daß** die Pergolid-haltige Matrixmasse Pergolidmesylat enthält.

4. TTS nach Ansprüchen 1 - 3, **dadurch gekennzeichnet, daß** die Pergolid-haltige Matrixmasse einen oder mehrere lipophile Hautpenetrationsenhancer und/oder einen oder mehrere Weichmacher enthält.

5. TTS nach Anspruch 4, **dadurch gekennzeichnet, daß** die Pergolid-haltige Matrixmasse Propylenglykolmonolaurat als Hautpenetrationsenhancer enthält.

6. TTS nach Anspruch 4, **dadurch gekennzeichnet, daß** die Pergolid-haltige Matrixmasse Zitronensäuretriethylester oder/und Zitronensäuretributylester als Weichmacher enthält.

7. TTS nach Ansprüchen 1 - 6, **dadurch gekennzeichnet, daß** die Trägerfolie matrixseitig eine Metalldampf- oder Oxidbeschichtung aufweist.

8. Verfahren zur Herstellung eines Transdermalen Therapeutischen Systems (TTS) zur transcutanen Verabreichung von Pergalid nach Ansprüchen 1 bis 7, **dadurch gekennzeichnet, daß** eine homogene, bis zu 150°C heiße Polymerschmelze, bestehend aus ammoniogruppenhaltigem (Meth)acrylatcopolymeren oder einer Mischung aus aminogruppenhaitigem (Meth)acrylatcopolymeren mit carboxylgruppenhaltigem (Meth)acrylatpolymeren, 10 bis 50 Gew.-% Propylenglykol, bis zu 5,0 Gew.-% Pergolid oder einem pharmazeutisch unbedenklichen Salz hiervon (berechnet als Pergolidbase) sowie gegebenenfalls einem oder mehreren Hautpenetrationsenhancerln und/oder einem oder mehreren Weichmacher/n kontinuierlich in einer Dicke von 0,02 - 0,4 mm auf einen Träger beschichtet, das erhaltene 2-Schichtlaminat mit einer Deckschicht versehen wird und hierauf ein größeres wirkstofffreies Pflaster zur Fixierung des TTS auf der Haut aufgebracht wird.

## Claims

1. Transdermal therapeutic system (TTS) for the transcutaneous administration of pergolide over several days with a means for sticking the TTS to the skin, **characterized in that** the TTS contains a pergolide-imbued matrix mass in the form of a layer which contains an ammonio-group (meth)acrylate copolymer or a mixture of amino-group (meth)acrylate copolymer and carboxyl-group (meth)acrylate polymer, 10 to 50 %wt of propylene glycol, and up to 5.0 %wt of pergolide or a pharmaceutically innocuous salt thereof (calculated as base) and is surrounded, except for its release surface at the application point, by a larger plaster, devoid of active substance, for sticking to the skin.

2. TTS according to Claim 1, **characterized in that** the matrix mass containing the active substance contains 0.5 to 2 %wt of pergolide or a pharmaceutically innocuous salt thereof (calculated as base).

3. TTS according to Claims 1 - 2, **characterized in that** the matrix mass containing the pergolide contains pergolide mesylate.

4. TTS according to Claims 1 - 3, **characterized in that** the matrix mass containing the pergolide contains one or more lipophile skin penetration enhancers and/or one or more softeners.

5. TTS according to Claim 4, **characterized in that** the matrix mass containing the pergolide contains propylene glycol monolaurate as skin penetration enhancer.

6. TTS according to Claim 4, **characterized in that** the matrix mass containing the pergolide contains citric acid triethyl ester or/and citric acid tributyl ester as softener.

7. TTS according to Claims 1 - 6, **characterized in that** the carrier foil has a metallized or oxide coating on the matrix side.

8. Method for the manufacture of a Transdermal Therapeutic System (TTS) for the transcutaneous administration of pergolide according to Claims 1 to 7, **characterized in that** a carrier is coated continuously with a 0.02 - 0.4 mm thick layer of a homogeneous polymer melt heated to a temperature of up to 150°C and consisting of ammonio-group (meth)acrylate copolymer or a mixture of amino-group (meth)acrylate copolymer with carboxyl-group (meth)acrylate polymer, 10 to 50 %wt of propylene glycol, and up to 5.0 %wt of pergolide or a pharmaceutically innocuous salt thereof (calculated as pergolide base) plus if required one or more skin penetration enhancer(s) and/or one or more softener(s), and the two-layer laminate obtained is provided with a cover layer and is then overlaid by a larger plaster, devoid of active substance, for sticking the TTS to the skin.

## Revendications

1. Système thérapeutique transdermique (STT) servant à l'administration transcutanée de pergolide pendant plusieurs jours avec un auxiliaire de fixation sur la peau pour le STT, ***caractérisé en ce que*** le STT contient une masse matricielle en forme de couche contenant du pergolide, qui contient un copolymère de (méth)acrylate contenant des groupes ammonio ou un mélange d'un copolymère de (méth)acrylate contenant des groupes amino et d'un polymère de (méth)acrylate contenant des groupes carboxyle, 10 à 50 % en poids de propylène-glycol, jusqu'à 5 % en poids de pergolide ou un sel de celui-ci pharmaceutiquement inoffensif (calculé en tant que base) et celui-ci est entouré, à l'exception de sa surface de libération sur le site d'application, d'un pansement plus grand dépourvu de substance active pour la fixation sur la peau.

2. STT selon la Revendication 1, ***caractérisé en ce que*** la masse matricielle contenant des substances actives contient de 0,5 à 2 % en poids de pergolide ou d'un sel de celui-ci pharmaceutiquement inoffensif (calculé en tant que base).

3. STT selon les Revendications 1 et 2, ***caractérisé en ce que*** la masse matricielle contenant du pergolide contient du mésylate de pergolide.

4. STT selon les Revendications 1 à 3, ***caractérisé en ce que*** la masse matricielle contenant du pergolide contient un ou plusieurs stimulateurs de pénétration transcutanée lipophiles et/ou un ou plusieurs plastifiants.

5. STT selon la Revendication 4, ***caractérisé en ce que*** la masse matricielle contenant du pergolide contient du monolaurate de propylène-glycol en tant que stimulateur de pénétration transcutanée.

6. STT selon la Revendication 4, ***caractérisé en ce que*** la masse matricielle contenant du pergolide contient du triéthylester d'acide citrique et/ou du tributylester d'acide citrique comme plastifiant.

7. STT selon les Revendications 1 à 6, ***caractérisé en ce que*** la feuille support présente du côté de la matrice un revêtement d'oxyde ou de vapeur métallique.

8. Procédé de fabrication d'un système thérapeutique transdermique (STT) pour l'administration transcutanée de pergolide selon les Revendications 1 à 7, ***caractérisé en ce qu***'une masse fondue de polymères homogènes à une température allant jusqu'à 150°C, composée de copolymère de (méth)acrylate contenant des groupes ammonio ou un mélange d'un copolymère de (méth)acrylate contenant des groupes amino et d'un polymère de (méth)acrylate contenant des groupes carboxyle, 10 à 50 % en poids de propylène-glycol, jusqu'à 5 % en poids de pergolide ou un sel de celui-ci pharmaceutiquement inoffensif (calculé en tant que base) ainsi que, le cas échéant, un ou plusieurs stimulateurs de pénétration transcutanée lipophiles et/ou un ou plusieurs plastifiants est enduite en continu avec une épaisseur de 0,02 - 0,4 mm sur un substrat, le stratifié à 2 couches obtenu est muni d'une couche de couverture et un pansement adhésif plus grand dépourvu de substance active est ensuite ajouté pour la fixation du STT sur la peau.
